# EUROPÄISCHE PATENTSCHRIFT

(11) **EP 2 190 498 B1**
(45) Veröffentlichungstag und Bekanntmachung des Hinweises auf die Patenterteilung: **10.07.2013**
(21) Anmeldenummer: 08787309.7
(22) Anmeldetag: 19.08.2008
(51) Int. Cl.: A61M 1/16, A61M 1/34

(54) **VORRICHTUNG ZUR EINSPARUNG VON DIAFILTRAT**
DEVICE FOR SAVING DIAFILTRATE
DISPOSITIF POUR FAIRE DES ÉCONOMIES DE DIAFILTRAT

(30) Priorität: 23.08.2007 DE 102007039939
(43) Veröffentlichungstag der Anmeldung: 02.06.2010
(73) Patentinhaber: Albutec GmbH, 18057 Rostock (DE)
(72) Erfinder: STANGE, Katrin, 18059 Rostock (DE)
(74) Vertreter: Garrels, Sabine
(86) Internationale Anmeldenummer: PCT/EP2008/060827
(87) Internationale Veröffentlichungsnummer: WO 2009/024566

(56) Entgegenhaltungen:
- WO-A-95/04560
- WO-A-03/041764
- WO-A-2005/044339
- US-A1- 2004 182 783

## Beschreibung

Der Verbrauch von steril verpackten Hemofiltrationssubstituatlösungen stellt den größten einzelnen Kostenfaktor bei kontinuierlichen Behandlungsverfahren dar. Zudem suggerieren jüngste Daten ein verbessertes Ergebnis bei höheren Diafiltratraten. Die vorliegende Erfindung beschreibt eine Vorrichtung zur Einsparung von Diafiltrat durch partielle Regeneration unter Verwendung von Adsorbern.

### Stand der Technik

Zur Darlegung des Standes der Technik sollen zu Beginn spezielle Begriffe definiert werden, die für die vorliegende Erfindung teils spezifisch sind.

**Komplexes biologisches Flüssigkeitskompartiment (A):** Komplexe biologische Flüssigkeitskompartimente bestehen aus einem oder mehreren miteinander kommunizierenden Flüssigkeitsra(e)um(en). Stoffkonzentrationsverläufe in komplexen biologischen Flüssigkeitskompartimenten sind durch Bildungs-, Verteilungs-, Umwandlungs- und Ausscheidungsmechanismen gekennzeichnet und verlaufen für verschiedene Substanzen (z.B. der Substanzgruppen X oder Y) unterschiedlich.

Ein Beispiel für ein komplexes biologisches Flüssigkeitskompartiment sind Bioreaktoren, zum Beispiel zur Nutzung des Stoffwechsels von Leberzellen. In diesem Fall wäre ein Flüssigkeitsraum das Reaktormedium und ein zweiter das Zellinnere der Leberzellen, wobei beide Räume miteinander über die Zellmembranen kommunizieren. Enzymatische Prozesse der Zelle und Verteilungsprozesse beeinflussen Konzentrationsverläufe von Substanzen im Reaktomedium. Ein weiteres Beispiel für ein komplexes biologisches Flüssigkeitskompartiment (A) ist der Blutkreislauf des Menschen.

**Membrandialyse/-filtration:** Unter Membrandialyse/-filtration wird ein im erweiterten Sinn kombiniertes Verfahren zur Kontrolle der Konzentrationen von Substanzen in einem komplexen Flüssigkeitskompartiment (A) verstanden, in denen die Flüssigkeit eines Raumes an einer porösen Membran entlang geleitet wird und entlang der Gegenseite eine Spülflüssigkeit (B) geführt wird, welche die in Flüssigkeit (A) unerwünschten Substanzen NICHT enthält und so diese Substanzen bei Porengängigkeit entsprechend einem Konzentrationsgefälle in das Kompartiment B durch Diffusion gelangen können (Dialyse). Dieser Prozess kann kombiniert werden mit einem konvektiven Transport, bei dem auch ein Flüssigkeitsstrom (crossflow) aus A in B durch Druckgefälle appliziert wird, der Substanzen auch konvektiv durch die Membran transportiert, wobei der das Kompartiment (A) verlassende (filtrierte) Teil (Filtrat) durch eine Substitutionslösung (Substituat) ganz oder auch nur teilweise ersetzt werden kann. Mit dem kombinierten Begriff der Membrandialyse/-filtration wird in dieser Anmeldung auch jede mögliche Kombination aus beiden Verfahren erfasst, wobei im Extremfall auch ausschließlich Filtration oder ausschließlich Dialyse appliziert wird.

Spüllösungskompartiment (B): ist im Sinne der vorliegenden Erfindung bei einer Membrandialyse/-filtration das von dem komplexen biologischen Flüssigkeitskompartiment (A) durch die Membran abgeteilte gegenüberliegende Flüssigkeitskompartiment, in dem sich Dialysat, Filtrat oder eine Kombination von beiden befindet.

Dialysat: Als Dialysat wird die in der Begriffserklärung "Membrandialyse/- filtration" verwendete Spülflüssigkeit bezeichnet, die der Membran auf der Spüllösungsseite (B) zur Stoffentfernung zugeleitet wird.

Substituat: Als Substituat wird die in der Begriffserklärung "Membrandialyse/-filtration" verwendete Substituatlösung bezeichnet, die bei Filtration als Flüssigkeitsersatz der Seite (A) dient.

Diafiltrat: Als Diafiltrat wird das in der Begriffserklärung "Membrandialyse/- filtration" die Spülseite (B) der Membran verlassende, also nach einem Reinigungszyklus mit unerwünschten Molekülen angereichertes Flüssigkeitsgemisch aus "Filtrat" und "Dialysat" bezeichnet.

**Nettodurchsatz an Dialysat/Substituat:** Der "Nettodurchsatz" an Diafiltrat ist der nach einmaliger Passage am Membranfilter verworfene, also nicht einem Reinigungszyclus und der Wiederverwendung zugeführte Anteil des Diafiltrats.

**Regenerationskreislauf (RKL):** Der Regenerationskreislauf ist eine in den Beispielen und den Ansprüchen näher beschriebene Vorrichtung, die z.B. durch Filtration, Adsorption oder biologische Reinigungsprozesse Stoffe der Substanzgruppe Y aus dem Diafiltrat wieder abreichert, aber nicht der Substanzgruppe X.

**Substanzgruppe X:** Hiermit wird in dieser Erfindung eine krankmachende Substanzgruppe zusammengefasst, die durch den Regenerationskreislauf (RKL) nicht direkt abgereichert werden kann, weil dieser keine Rückhalte- bzw. Adsorptionskapazität für diese Gruppe X besitzt. Die unter Gruppe X zusammengefasste Substanzgruppe kann durch Dialyse oder Filtration entfernt werden.

**Substanzgruppe Y:** Hiermit wird eine krankmachende Substanzgruppe zusammengefasst, die durch den Regenerationskreislauf (RKL) abgereichert werden kann, da dieser für Y eine Rückhalte- bzw. Adsorptionskapazität hat.

Die Reinigung komplexer Stoffgemischsysteme z.B. Blut durch Membrandialyse/-filtration birgt heute oft den unnötig hohen Verbrauch von Dialysat oder Substituat, da deren Flussrate so eingestellt werden muss, dass die am schnellsten anflutende pathophysiologisch relevante Substanz noch effektiv genug entfernt bzw. deren Konzentration im Kompartiment A kontrolliert wird.

In komplexen biologischen Flüssigkeitskompartimenten, wie z.B. in Bioreaktoren zur Kultivierung von Leberzellen, kann dieses zum Beispiel der durch den Krebszyclus gebildete Harnstoff sein. Dieser könnte aus dem Reaktomedium durch Diafiltration entfernt werden. Auch im Blutkreislauf von Patienten mit Nierenschäden kann sich Harnstoff ansammeln.

Besonders in intensivmedizinischen Anwendungen führt dieses zu einem oft unnötigen Verbrauch von preisintensiven steril abgepackten Dialysat-und Substituatlösungen.

In Abhängigkeit davon wie hoch die Entfernungsrate der pathophysiologisch relevanten Substanz bei jeweils eingestellter Flussrate ist, wird auch die tägliche Behandlungszeit festgelegt. Ist die Entfernungsrate in Folge geringer Flussraten gering, muss entsprechend die Behandlungszeit verlängert werden, was zu verlängerter Antikoagulation (z.B. Heparin oder Citrat) zwingt, welche Nebenwirkungen (z.B. Blutungen oder Alkalose und Hypernaträmie) haben kann.

Die extrakorporale Blutreinigung durch Dialyse und Filtration basiert auf dem diffusiven (Dialyse) und/oder konvektiven (Diafiltration) Transport porengängiger Moleküle aus dem Blut oder Plasma durch eine poröse Membran in ein Spüllösungs- (Diafiltrat) Kompartiment hinein.

Bei der Dialyse und der Filtration braucht man also eine saubere Lösung die frei von unerwünschten Substanzen sein sollte, die entweder als Substituat bei der Filtration oder Dialysat bei der Dialyse gebraucht wird. Andererseits sollten Substanzen die der biologischen Flüssigkeit nicht entzogen werden dürfen (wie im Fall von Blut z.B. Glucose) in dem Substituat bzw. Dialysat in gleicher Konzentration enthalten sein wie in besagter Flüssigkeit. In der verbreiteten Dialyse werden diese Flüssigkeiten in der Regel online aus Konzentraten und Reverese-Osmose Wasser online angemischt, wozu eine komplexe Technologie (Wasseraufbereitungsanlagen, Dialysemaschinen) notwendig ist. Wegen der hohen technologischen Komplexizität ist daher Fachpersonal (Dialyseschwestern) und auch die Logistik eines Wasserzuflusses notwendig.

Zum bekannten Stand der Technik gehören Systeme mit geschlossenem Dialysatkreislauf ohne kontinuierliche Zufuhr von Dialysat und/oder Substituat.

Im BioLogic DT System, wird eine kleine geschlossene Menge Dialysatreservoir im Rezirkulationsmodus angewandt. Das Reservoir wird regeneriert durch eine Suspension von Ionenaustauschern und einer relativ feinporösen Aktivkohle. Es wird kein kontinuierlicher Dialysatstrom eingesetzt, welcher die Abreicherung von durch den Regenerationskreislauf nicht entfernbaren Substanzen ermöglicht. Das System spart zwar Dialysat, hat sich aber besonders zur Kontrolle des Harnstoff und Ammoniakspiegels nicht bewährt. Es wird keine differenzierte Teilregeneration und Teilwiederverwendung von Dialysat oder von Substituat ermöglicht.

Im Ready System wird eine kleine geschlossene Menge Dialysatreservoir im Rezirkulationsmodus angewandt. Das Reservoir wird regeneriert durch einen aufwendigen Prozess, der die Zersetzung von Harnstoff in toxisches Ammoniak durch ein Enzym (Urease) benötigt welches sekundär durch Zirkoniumphosphat chemisch umgewandelt werden muss.

Das System spart zwar Dialysat, durch den hohen Anfall an Ammoniak durch die Urease wird aber eine effektive Entfernung von Ammoniak aus dem Patientenblut kompromittiert.

Es wird auch gar kein kontinuierlicher Dialysatstrom eingesetzt, welcher die Abreicherung von durch den Regenerationskreislauf nicht entfernbaren Substanzen ermöglicht, wobei darauf hinzuweisen ist, dass viele der unerwünschten Substanzen in komplexen biologischen Flüssigkeitskompartimenten noch nicht bekannt sind.

Beim Redy System, wo eine 100-prozentige Regeneration von Dialysat oder von Substituat durchgeführt wird, besteht also die Gefahr der Akkumulation von durch den Regenerationskreislauf nicht entfernbaren Substanzen.

Im Genius System wird eine große geschlossene Menge Dialysatreservoir im Rezirkulationsmodus angewandt. Die Kapazität der Entgiftung wird hier durch das ausgesprochen hohe Volumen des Dialysats (bis zu 80 Liter) bereitgestellt. Es wird keine differenzierte Teilregeneration und Teilwiederverwendung von Dialysat oder von Substituat ermöglicht. Ist die Konzentration im Dialysat auf das Blutniveau angestiegen, muss das System gewechselt werden.

Kombinierte Dialyse und Adsorptionen (Renaltech, etc.) werden in Reihe und im direkten Blutkontakt vorgestellt, und sind damit wenig bioverträglich und auch nicht unabhängig voneinander einstellbar. Hierbei werden Adsorber im direkten Blutkontakt angewandt, um schwer dialysierbare Substanzen durch Adsorption aus dem Blut zu entfernen.

Verfahren, bei denen Adsorbentien in Verbindung mit einem Plasmapheresefilter eingesetzt werden (Plasmapherese, Prometheus), erlauben in der Regel keine hohen transmembranalen Flüsse und bergen die Gefahr des Verlustes von wichtigen Proteinen oder anderen Wertstoffen an den Adsorbern.

Das MARS-Verfahren (EP 0615780 B1) dient der kombinierten Entfernung von wasserlöslichen und proteingebundenen Substanzen. Seine Besonderheit besteht darin, dass zunächst das biologische Kompartiment (A), meist Blut, an der engporigen, proteinundurchlässigen (Blut-)Seite einer asymmetrischen Dialysemembran entlang geleitet wird, die mit Proteinen beschichtet ist, welche eine Bindung zu proteinaffinen Toxinen haben. Auf der gegenüberliegenden Spüllösungsseite (B) wird eine Dialysatflüssigkeit mit einem darin gelösten Protein mit Bindungsfähigkeit für proteinaffinen Toxinen entlanggeleitet, welches die grobporige Spüllösungsseite der Membran betreten kann und nahe an die der Blutseite zugewandten proteinundurchlässige Grenzfläche (auch Thin Layer) der asymmetrischen Dialysemembran zu erreichen vermag. Es hat sich gezeigt, dass über die kurze Breite des Thin Layers eiweißgebundene Toxine von der biologischen Lösung zu den Dialysatproteinen überzugehen vermögen und dann mit dem proteinhaltigen Dialysat abtransportiert werden können. Zusätzlich werden wasserlösliche porengängige Moleküle entsprechend Konzentrationsgefälle ausgetauscht.

Da diese Proteine teuer sind, wird das proteinhaltige Dialysat nach Benutzung zunächst durch einen in Reihe geschalteten zweiten Dialysator durch ein sekundäres Dialysat dialysiert und anschließend in Reihenschaltung das Protein durch Adsorber von den proteinaffinen Molekülen befreit, so dass das primäre, proteinhaltige Dialysat wieder verwendet werden kann. Eine differenzierte Regeneration der Dialysatlösung im Interesse der Einsparung von Dialysat findet nicht statt. Im Gegenteil wird durch die Anwendung eines sekundären Dialysatkreislaufes zur Entfernung dialysabler Substanzen die Effektivität des Dialyseprozesses gemindert, weshalb bei den publizierten kontrollierten Studien (Hemann et al. Hepatology 2002) welche eine klinische Effektivität gezeigt haben, meist Dialysatflüsse von 500 ml/min im sekundären Dialysat nötig waren. Eine differenzierte Teilregeneration und Teilwiederverwendung von Dialysat- bzw. Substituatflüssigkeit zur Einsparung von Dialysat oder Substituat ist durch das MARS-Verfahren nicht beschrieben.

In WO 2005/044339 A wird eine weitere Vorrichtung zur Blutreinigung durch Dialyse offenbart, wobei eine gezielte, sogenannte "Backfiltration" zum Einsatz kommt. Dabei wird konvektiv aus dem Blut Filtrat in ein Filtratkompartiment "gedrückt" welches dem Blut wiederum als "Backfiltrat" zurückgegeben wird. Die Backfiltration kann dabei über einen oder mehrere Dialysatoren gestaltet werden. Giftstoffe werden direkt durch den Filtrat- bzw. Recyclingmechanismus gebunden bzw. entfernt.

WO 95/04560 A beschreibt eine Vorrichtung zur Inhibition von Tumorwachstum durch die extrakorporale Entfernung essentieller Aminosäuren, z.B. von Arginin. Es wird ein in sich geschlossener Rezirkulationskreislauf offenbart, in dem die Aminosäure durch Filtration in ein Filtrat überführt wird, wo sie durch verschiedene Mechanismen (z.B. Adsorption) direkt entfernt wird.

In der Patentveröffentlichung US 2004/182783 A1 wird primär die Entgiftung durch Hemofiltration mittels Prädilution beschrieben. Zusätzlich wird zur Elimination von Toxinen ein RKL beschrieben, der durch Filtrationsmechanismen Moleküle der in der Erfindung beschriebenen Substanzklasse X direkt entfernt.

Die Publikation WO 03/041764 A1 beschreibt ein Verfahren und eine Vorrichtung zur Regeneration von Dialysat durch eine Kombination von Enzymen und Adsorber.

### Darstellung der Erfindung

Aufgabe der Erfindung ist es, den Bedarf an Dialysat und/oder Substituat bei der Abreicherung einer Substanz der Klasse X (siehe Begriffsdefinitionen eingangs) dadurch zu senken, dass durch einen internen Regenerationskreislauf Substanzen der Klasse Y, die entweder Vorläufer oder Metabolite der Substanz X im komplexen biologischen Flüssigkeitskompartiment sind, mittels speziellen mikrostrukturierten Adsorbtions-/Filtrationsvorrichtungen aus dem Filtrat bzw. Dialysatverwurf entfernt werden, so dass Teile des Filtrats bzw. Dialysatverwurfs im Rahmen einer Rezirkulation mehrfach verwendet werden können während der Nettodurchsatz and Dialysat bzw. Filtrat so minimiert wird, dass die Konzentration ausgewählter Markersubstanzen der Klasse X im komplexen biologischen Flüssigkeitskompartiment vorgegebene Zielgrößen nicht überschreitet.

Aufgabe der Erfindung ist es auch, die Kontrolle der Konzentration einer Substanzgruppe X durch Hemodialyse und/oder Hemofiltration zu ermöglichen, so dass durch partielle Rezirkulation des Diafiltrats im Regenerationskreislauf der Nettodurchsatz von Diafiltrat geringer gehalten werden kann.

Erfindungsgemäß wird die Aufgabe gemäß der Vorrichtung nach Anspruch 1 gelöst.

Es wurde die überraschende Beobachtung gemacht, das z.B. die Kontrolle der Konzentrationen von Harnstoff in einer komplexen biologischen Flüssigkeit durch Dialyse dadurch besser gelingt, das durch partielle Regeneration des Dialysats über Adsorptions- und/oder Filtrationseinheiten Metabolite bzw. Vorläufer von Harnstoff entfernt werden, nicht aber der Harnstoff selbst.

Die Vorteile der Erfindung bestehen in einer besseren Kontrolle von Konzentrationen der Substanzklasse X in komplexen biologischen Flüssigkeitskompartimenten bei möglichst geringem Nettoverlust von sterilem Dialysat.

Damit werden nicht nur erheblich die Behandlungskosten reduziert, sondern auch der Aufwand beim Transport des Dialysats (in der Regel in schweren 4,5 I Beuteln geliefert) wird gesenkt.

Einer der größten Vorteile der Erfindung besteht jedoch in der Nachhaltigkeit intermittierender Diafiltrationsbehandlungen. Dadurch, dass durch Elimination der Substanzen der Gruppe Y auch die Nachbildung der Substanz der Gruppe X verzögert bzw. der biologische Abbau der Gruppe X verstärkt wird kann die Entfernung der Toxine auch über nachfolgende biologische Stoffwechselreaktionen "verlängert" nachwirken.

### Kurze Beschreibung der Abbildungen

Die Erfindung soll an den folgenden Ausführungsbeispielen näher erläutert werden. Hierzu zeigt

Figur 1 Harnstoffkinetik (1a) und Glutaminclearance (1 b) bei normaler CVVHD und bei erfindungsgemäßem Verfahren mit Regenerationskreislauf, welches auch in Figur 3 dargestellt ist,

Figur 2 Ammoniakverlauf in Plasma als komplexes biologisches Flüssigkeits-kompartiment bei Dialyse ohne Regenerationskreislauf und bei erfindungsgemäßen Aufbau entsprechend Figur 3,

Figur 3 ein Schaltbild für eine Dialyse mit Regenerationskreislauf,

Figur 4 ein Schaltbild für eine Filtration kombiniert mit Substitution mit Regenerationskreislauf wobei die Substitution entweder als "Postdilution" (4a) oder als "Prädilution" (4b) erfolgen kann,

Figur 5 ein Schaltbild für eine kombinierte Dialyse und Filtration (Diafiltration) mit Substitution, wobei sowohl Teile des Dialysats als auch des Substituates aus durch Regenerationskreislauf regeneriertem Filtrat bestehen.

### Ausführung der Erfindung

**Ausführungsbeispiel 1:** Substanzgruppe X Harnstoff, Substanzgruppe Y Glutamin und/oder Ammoniak

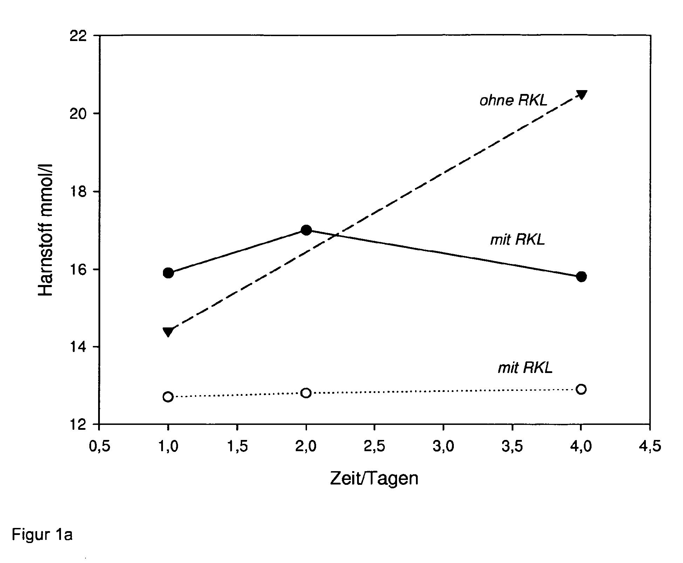

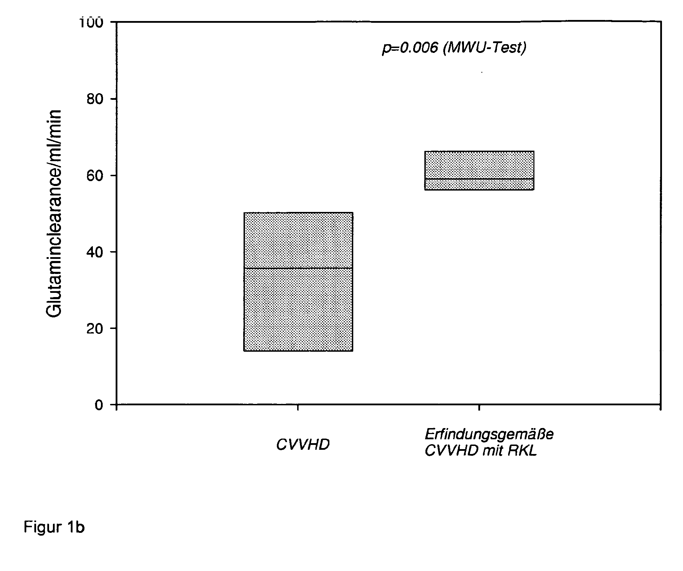

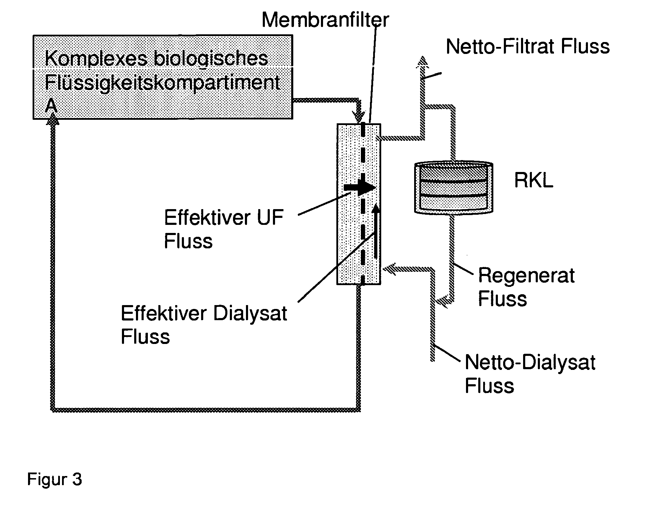

Die Erfindung beruht in einem Ausführungsbeispiel entsprechend Figur 3 darauf, dass unter Verwendung eines Aktivkohle-Adsorbers OHNE Aufnahmeleistung für Harnstoff zum Teilrecycling einer sterilen Bicarbonatdialysatflüssigkeit (Fluss 150 ml/min) bei einem CVVHD Behandlungsmodus mit langsamen Blutfluss (150ml) und mäßiger Netto-Dialysatflussrate (50ml) und damit geringer Harnstoff-Clearance (50 ml/min) trotz kurzer Behandlungszeit (<8h) bei dialysepflichtigen Patienten mit akutem Nierenversagen mit einem Körpergewicht von 60 kg, also einem Kt/V Verhältnis (Clearance mal Zeit geteilt durch Körpervolumen) von nur 0,48 (sollte normalerweise über 1 sein) über eine Behandlungszeit von 3 Tagen ein stabiler und akzeptabler Serumharnstoffspiegel beibehalten wurde, während bei vergleichbaren Patienten in einem identischen Behandlungszeitraum von 3 Tagen bei gleichem Blut- und Dialysatfluss und vergleichbaren Behandlungszeiten mit CVVHD ohne ein Teilrecycling ein signifikanter Anstieg des Harnstoffs zu beobachten war (Figur 1 a).

Potentielle Co-Variaten der Harnstoffkinetik, wie die endogene Harnstoffbildung durch Katabolie oder die renale Harnstoffclearance waren ebenfalls vergleichbar und konnten als Ursache für diese überraschende Beobachtung ausgeschlossen werden.

Bei einer eingehenden Untersuchung des Phänomens hat sich überraschender Weise gezeigt, dass das interne Recycling der sterilen Bicarbonat-Dialysatlösung zwar zu keiner Entfernung von Harnstoff als Zielsubstanz führte (da Aktivkohle in der von den Erfindern angewandten Form bekannterweise keine wesentlich Harnstoffbindung ermöglicht), jedoch konnte festgestellt werden, dass im Teil-Recycling-Modus die Glutaminclearance signifikant höher war (Figur 1 b) als bei herkömmlicher CVVHD, obwohl bei beiden Verfahren die Nettodurchsatzrate für Bicarbonat-basiertes Diafiltrat identisch war.

Bei weiterreichenden Untersuchungen konnte gezeigt werden, dass die gute Bindungsfähigkeit für Glutamin auch durch eine gezielte Steuerung der Mikrostruktur eines Aktivkohlefestbettadsorbers steigern lässt, wenn die Perfusionskanäle in einem speziellen Weitenbereich eingestellt werden (10 nm bis 100 Mikrometer). Hierdurch wird eine effektive, nahezu 100%-ige "Single pass" Abreicherung des Glutamins im Durchstrom erreicht, wodurch der Konzentrationsgradient für Glutamin über die Membran maximal ist. Beim BioLogic Systems z.B. ist im Gegensatz zu solchen mikrostrukturierten "Festbettadsorbern" der Adsorber als "Fluid Bed" Adsorber im Dialysat organisiert, was zu einer wesentlichen Vergrößerung der Diffusionsabstände zwischen den einzelnen Adsorberpartikeln führt. Daher ist hier die Glutaminclearance erheblich eingeschränkt, der Konzentrationsgradient für Glutamin über die Membran ist viel höher. Beim BioLogicDT Verfahren sind also durch Verwendung von feinporigen Aktivkohlepartikeln in einer Suspension einerseits die Diffusionsstrecke Moleküle weit, andererseits der Zugang zum Aktivkohleinneren für größere Moleküle durch den hohen Anteil von Mikroporen eingeschränkt. Dieses in Kombination mit der Tatsache, dass gar kein Nettodurchsatz von Diafiltrat ermöglicht wird hat dazu geführt, dass unter der Behandlung mit der Biologic DT keine effektive Senkung bestimmter wasserlöslicher Stoffe der Klasse X beobachtbar war, weshalb das System auch vom Markt verschwand. Ein Teilrecycling des Dialysats/Filtrats mit einem internen Regenerationskreislauf mit geeigneter Aktivkohle mit Perfusionskanälen von 10nm bis 100 Mikrometern führt bei Anwendung einer erfindungsgemäßen Vorrichtung jedoch zu einem selektiven Abfall von Stickstoffquellen, die ihrerseits eine sekundäre Verminderung der Harnstoffbildung zur Folge haben.

Entweder zusätzlich oder in Kombination mit einem Aktivkohlefilter kann auch ein spezieller Adsorber zur Entfernung von Ammoniak eingesetzt werden, der zusätzlich eine Stickstoffquelle entfernt und damit die Kontrolle der Harnstoffkonzentration in komplexen biologischen Systemen erlaubt.

**Ausführungsbeispiel 2:** Substanzgruppe X Ammoniak, Substanzgruppe Y Glutamin

Nach dem Stand des Wissens kann beispielsweise Ammoniak durch Dialyse nur dann effektiv entfernt werden, wenn mindestens ein Blutfluss von über 200 ml und ein Dialysatfluss von über 500 ml eingestellt wurde bei einer Membranoberfläche von 1.3 qm (Cordoba et al. 1996).

Es hat sich gezeigt, dass bei Verwendung eines internen Regenerationskreislaufes (RKL) der einen Adsorber auf der Basis von Aktivkohle enthält, eine effektive Senkung des Ammoniaks auch bei erheblich geringerem Nettodurchsatz von Dialysat möglich ist, was auf der selektiven Elimination von Glutamin im Verhältnis zu Glutamat beruht.

Hierzu wurde in einem in vitro Aufbau folgende Experimente durchgeführt:

In Anlehnung des von Cordoba et al. beschriebenen Modellaufbaus für komplexe biologische Flüssigkeitskompartimente wurde ein Patientenmodell durch einen Liter Plasma (Versuche A, B und C) bzw. einen Liter 5%ige Humanalbuminlösung (Versuch D) simuliert die mit 53 mg Ammoniak versetzt wurden, wobei die Nachverteilung aus dem Gewebe dadurch simuliert wurde, dass kontinuierlich eine Lösung mit 1350 mg/l Ammoniak in das Patientenmodell mit einer Geschwindigkeit von 90ml/min zudosiert wurde. Versuch D wurde mit Albumin als Patientenmodell durchgeführt, um zu demonstrieren, dass das Fehlen von Enzymen, die im Plasma in Spuren vorkommen (Z.B. Gamma-Glutamyltransferase) zu einer veränderten Kinetik von Ammoniak im Patienten unter Behandlung führt.

Anschließend wurde im Versuch B das Patientenmodell mit einem 1,7 qm großen Standarddialysator dialysiert (Patientenflußrate 250 ml/min, Nettodialysatdurchfluss 50 ml/min) und im Versuch C unter ansonsten identischen Bedingungen ein Regenerationskreislauf mit 250 ml/min eingebaut, der über einen Aktivkohlefilter regeneriert wurde. Der Versuch A wurde ohne aktive Entgiftung durchgeführt, um die natürliche Akkumulation im Patientenmedium zu dokumentieren. Es wurde der Konzentrationsverlauf für Ammoniak im Patientenmodell am Beginn und nach 10, 20, 30, 45, 60, 90 und 120 Minuten gemessen. Zusätzlich wurde über die gleiche Zeit in den Versuchen B und C durch Probeentnahmen vor und nach dem Dialysator die Ammoniakclearance nach der Formel

Clearance=Blutfluss x ((Eingangskonzentration-Ausgangskonzentration)/Eingangskonzentration)

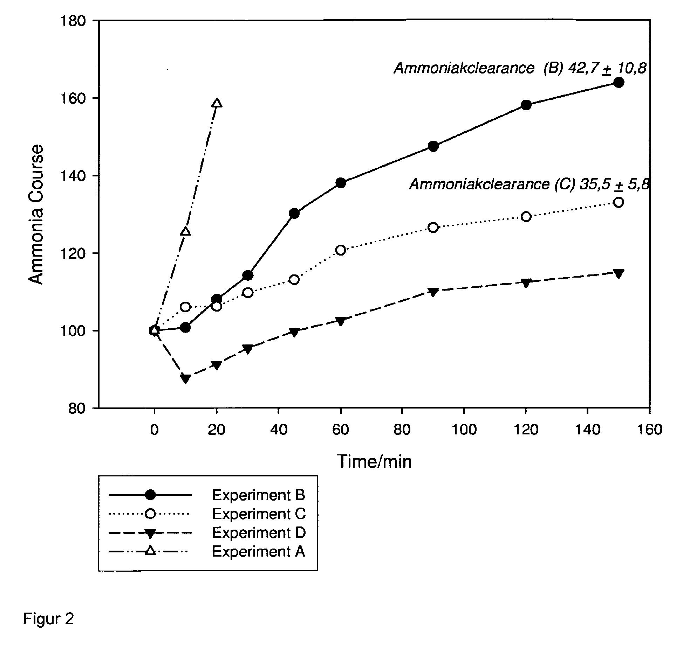

errechnet, um den Konzentrationsverlauf im Patienten im Zusammenhang mit der Clearance interpretieren zu können. Gleichfalls wurde im Experiment C die Ammoniakkonzentration zu den gleichen Zeitpunkten vor und nach dem Aktivkohlefilter gemessen. In der Figur 2 wird der Patientenkonzentrationsverlauf der Experimente A-D über der Zeit dargestellt, wobei deutlich wird, dass im Vergleich zur normalen Dialyse (Versuch B) durch einen Regenerationskreislauf über den Kohlefilter (Versuch C) der Ammoniakanstieg signifikant (p=0.036 im Wilcoxon Test für gepaarte Stichproben) gesenkt werden konnte. Dabei konnte durch die Bestimmung der Clearancewerte bewiesen werden, dass dieser verminderte Anstieg NICHT durch eine direkte größere Entfernung von Ammoniak durch den Dialysator erfolgte, da eine Ammoniakentfernung durch den Aktivkohlefilter nicht nachweisbar war und die Clearance im Versuch C sogar niedriger (35,5±5,8 ml/min) als im Versuch B (42,7±10,8 ml/min) war (p=0,09). Der im Vergleich zum Plasmaversuch B durchgeführte Dialyseversuch der Albuminlösung (D) zeigte wiederum nur einen moderaten Ammoniakanstieg.

Die Ammoniak-Verläufe der Experimente B, C und D sind in Figur 2 dargestellt, der Basisanstieg (A) ist wegen Achsenbegrenzung nur über die ersten 30 min dargestellt.

Damit ist erwiesen, dass im Plasma als komplexe biologische Flüssigkeit enzymatische Prozesse stattfinden, welche eine zusätzliche Generation von Ammoniak erzeugen (z.B. die Gamma-glutamyltransferase vermittelte Aufspaltung von Glutamin), die durch die Entfernung von Substrat (z.B. Glutamin) gehemmt werden.

**Ausführungsbeispiel 3:** Substanzgruppe X Stickstoffmonoxid, Substanzgruppe Y Arginin

Nach dem heutigen Stand des Wissens ist eine aktive Entfernung durch extrakorporale Blutreinigungen für die Verfügbarkeit von Stickstoffmonoxid am Wirkort (z.B. im Gewebe oder in der Mikrozirkulation) nicht ausschlaggebend, da es sich beim Stickstoffmonoxid um ein äußerst kurzlebiges Radikal handelt, welches seine Wirkung lokal entfaltet und dann sofort zerfällt.

Durch eine Vorrichtung zum effektiven Teilrecycling von CVVHD Dialysat durch spezielle mikrostrukturierte Adsorber zur Erhöhung der Arginineclearance wird jedoch parallel eine verbesserte Kontrolle des plasmatischen NO-Spiegels möglich.

Dabei wird während einer CVVHD Behandlung mit einem effektiven Blutfluss von 120 ml/min und einem Dialysatfluss von 40 ml/min in einer Ausführung ein Regenerationskreislauf von 100 ml/min mit einem mikrostrukturierten Aktivkohlefilter (maximale Perfusionskanalweite 100 um) aufgebaut, während parallel eine CVVHD ohne Regenerationskreislauf eingesetzt wird.

In dem einen Fall konnte eine Senkung des NO Spiegels (gemessen am Nitrate/Nitrite Spiegel) von 112 auf 26 umol/l innerhalb von 24 h bzw. von 108 auf 24 umol/l in 16 h erreicht werden, während innerhalb von 48 h normaler CVVHD der NO Spiegel sogar von 24 auf 125 umol/l wieder anstieg. Die messbare Entfernung von NO in das Dialysat hinein war jedoch nicht voneinander signifikant unterschiedlich. Hingegen war jedoch die Argininclearance im Fall des Regenerationskreislaufes 72 ml/min ± 25 ml/min und im Fall des CVVHD Modus' 36 ± 3 ml/min (p<0.05).

**Ausführungsbeispiel 4:** Substanzgruppe X TNF alpha und Substanzgruppe Y IL1

Im Rahmen einer CVVHF wird der Verlauf von TNF und IL 1 beta zwischen einem Teil Recycling System und einer conventionellen CVVHF verglichen. Die Blutflussrate war 150 ml/min, der Substituat/Filtratfluss bei der CVVHF 2,5 l/h. Als Filter wird ein hochpermeabler Filter verwendet (F50, Fresenius). Bei dem Rezirkulationsaufbau wird zusätzlich ein innerer Reislauf mit 150 ml/h im Dialysatmodus aufgebaut, ansonsten sind die Parameter identisch. IL1 beta und TNF alpha werden am Anfang und nach Ende der Behandlung im Blut gemessen. Zusätzlich wird die Ultrafiltration von TNF alpha in das Filtrat gemessen.

Die TNF alpha Ultrafiltration ist bei beiden Aufbauten nicht signifikant voneinander unterschiedlich. Dennoch ist im Rezirkulationsmodus eine Senkung des TNF alpha im Blut von 150 ± 90 auf 100 ± 40 pg/ml (p<0.05 im gepaarten t-test) zu messen, während bei normaler CVVHF keine signifikante Senkung zu verzeichnen ist. Parrallel kann jedoch beim Rezirkulationsmodus eine signifikante Senkung des IL 1 beta von 9±7 auf 6±6 pg/ml gemessen werden (p<0.05 im gepaarten t-test), was beim CVVHF Modus gleichfalls nicht signifikant ist.

In den Figuren 3 bis 5 werden verschiedene Schaltbilder aufgezeigt, um das Verfahren umzusetzen. Hierzu wird in Figur 3 ein Schaltbild für eine Dialyse mit Regenerationskreislauf gezeigt. Dargestellt ist ein für den Fachmann übliches Schaltungsmuster einer Dialyse. Das komplexe biologische Flüssigkeitskompartiment A (z.B. Blutkreislauf) wird mittels Schläuchen an einer Seite eines Membranfilters (wobei dieses ein Filter beliebiger Porenweite ohne besondere Einschränkungen mit Ausschlussgrenzen zwischen 5000 Dalton bis zu 500.000 Dalton sein kann) entlanggeleitet, während die Dialysat (Spül-) lösung auf der Dialysatseite entlang fließt. In Abhängigkeit der Bilanziereinrichtung kann zusätzlich auch ultrafiltriert werden (konvektiv mit Flüssigkeitsnettostrom durch die Membran in das Dialysat). Entscheidend ist die Aufspaltung des Diafiltrates in zwei Ströme, wovon einer verworfen wird und der andere über einen Regenerationskreislauf (RKL) wiederverwertet (Regenerat-Fluss), wobei die Substanzgruppe Y zurückgehalten wird, aber nicht die Substanzgruppe X, und dem frischen Dialysat (Netto-Dialysat) wieder beigemischt wird. Durch den Netto-Filtrat Fluss wird die Substanzgruppe X abtransportiert.

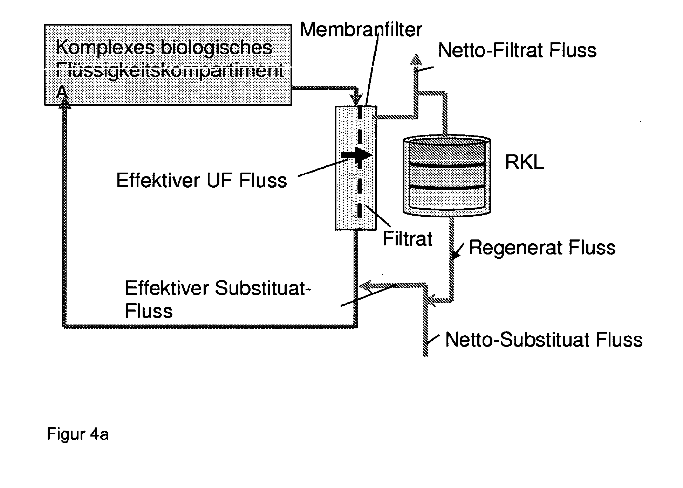

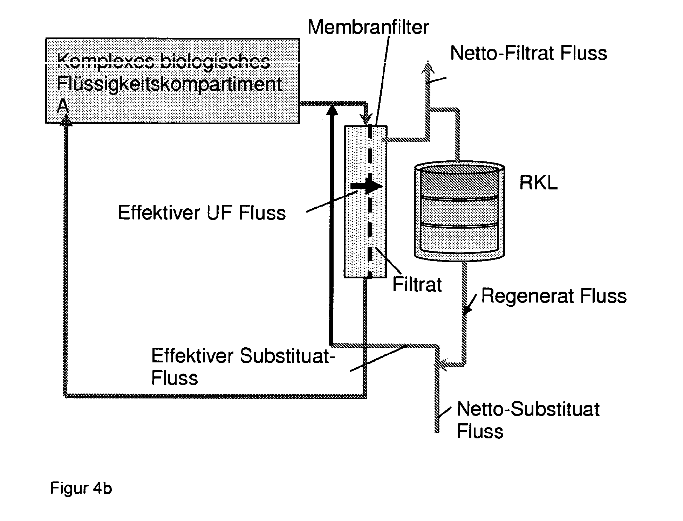

Ein Schaltbild für eine Filtration kombiniert mit Substitution mit Regenerationskreislauf wird in Figur 4 gezeigt, wobei die Substitution entweder als "Postdilution" (Figur 4a) oder als "Prädilution" (Figur 4b) erfolgen kann. Dargestellt ist ein für den Fachmann übliches Schaltungsmuster einer Filtration. Das Komplexe biologische Flüssigkeitskompartiment A wird mittels Schläuchen an einer Seite eines Membranfilters (wobei dieses ein Filter beliebiger Porenweite ohne besondere Einschränkungen mit Ausschlussgrenzen zwischen 5000 Dalton bis zu 500.000 Dalton sein kann) entlanggeleitet, während in Abhängigkeit der Bilanziereinrichtung ultrafiltriert wird, also konvektiv mit Flüssigkeitsnettostrom durch die Membran in das Filtratkompartiment. Der Flüssigkeitsverlust wird in Abhängigkeit der gewünschten Bilanz teilweise oder ganz durch Substitution mit Substituatlösung entweder als Postdilution (4a) oder Prädilution (4b) in das biologische Kompartiment A direkt ersetzt. Entscheidend ist die Aufspaltung des Filtrates in zwei Ströme, wovon einer verworfen wird und der andere über einen Regenerationskreislauf (RKL) wiederverwertet (Regenerat-Fluss), wobei die Substanzgruppe Y zurückgehalten wird, aber nicht die Substanzgruppe X, und dem Substituat (Netto-Substituat) beigemischt wird. Durch den Netto-Filtrat Fluss wird die Substanzgruppe X abtransportiert.

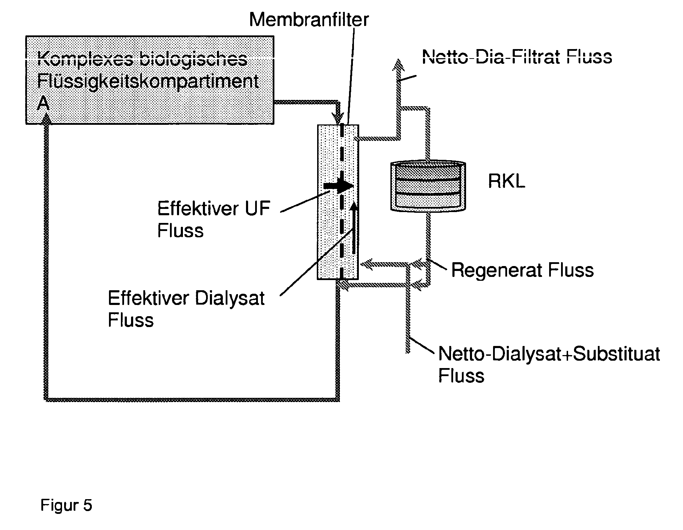

Ein Schaltbild für eine kombinierte Dialyse und Filtration (Diafiltration) mit Substitution zeigt Figur 5, wobei sowohl Teile des Dialysats als auch des Substituates aus durch Regenerationskreislauf regeneriertem Filtrat bestehen. Dargestellt ist ein für den Fachmann übliches Schaltungsmuster einer kombinierten Dialyse und Filtration. Das Komplexe biologische Flüssigkeitskompartiment A wird mittels Schläuchen an einer Seite eines Membranfilters (wobei dieses ein Filter beliebiger Porenweite ohne besondere Einschränkungen mit Ausschlussgrenzen zwischen 5000 Dalton bis zu 500.000 Dalton sein kann) entlanggeleitet, während die Dialysat (Spül-) lösung auf der Dialysatseite entlang fließt. In Abhängigkeit der Bilanziereinrichtung kann zusätzlich auch ultrafiltriert werden, also auch konvektiv mit Flüssigkeitsnettostrom durch die Membran in das Diafiltratkompartiment. Der Flüssigkeitsverlust wird zusätzlich in Abhängigkeit der gewünschten Bilanz teilweise oder ganz durch Substitution mit Substituatlösung in das biologische Kompartiment A direkt ersetzt. Entscheidend ist die Aufspaltung des Diafiltrates in zwei Ströme, wovon einer verworfen wird und der andere über einen Regenerationskreislauf (RKL) wiederverwertet (Regenerat-Fluss), wobei die Substanzgruppe Y zurückgehalten wird, aber nicht die Substanzgruppe X, und dem Dialysat und/oder Substituat (Netto-Dialysat und oder Netto-Substituat) beigemischt wird. Durch den Netto-Filtrat Fluss wird die Substanzgruppe X abtransportiert. Für den Fachmann sind hier alle Kombinationen aus Figur 3 und 4 nachvollziehbar.

## Patentansprüche

1. Vorrichtung zur Einsparung von Diafiltrat bei einer Dialysevorrichtung, bestehend aus Regenerationskreislauf, Pumpe und Filter **dadurch gekennzeichnet, dass**
an einen Diafiltratschlauch, der die Flüssigkeit eines Spüllösungskompartiments (B) aus einem Filter sammelt, ein 3-Wege-Anschluss angeordnet ist, durch den ein Teilstrom des Diafiltrats über eine separate Pumpe mit einem Regenerationskreislauf (RKL) in der Art verbunden ist, dass das Diafiltrat in zwei Ströme aufgespalten wird, wovon der eine Strom mit der Substanzgruppe X verworfen wird und der andere mit der Substanzgruppe Y über den Regenerationskreislauf (RKL) wiederverwertet wird, und dass die Substanzgruppe Y dem frichen Diafiltrat über einen 3-Wege-Anschluss wieder beigemischt wird.

2. Vorrichtung nach Anspruch 1 **dadurch gekennzeichnet, dass**
Netto-Dialysat und/oder Netto-Substituat beigemischt wird.

3. Vorrichtung nach Anspruch 1 oder 2 wobei der Regenerationsgreislauf mindestens, aber nicht unbedingt ausschließlich einen Adsorber auf Basis von Aktivkohle enthält.

4. Vorrichtung nach Anspruch 1 oder 2 wobei der Regenerationsgreislauf mindestens, aber nicht unbedingt ausschließlich einen Membranfilter enthält, der durch eine geringere Porengröße als der ein Flüssigkeitskompartment (A) prozessierende Membranfilter Stoffe aus dem RKL entfernt, die zuvor aus dem Flüssigkeitskompartment (A) in das Diafiltrat abgefiltert wurden.

5. Vorrichtung nach Anspruch 1 oder 2 wobei die Porenweite des Membranfilters die Passage von Glutamin erlaubt und der Regenerationsgreislauf mindestens, aber nicht unbedingt ausschließlich einen Adsorber auf Basis von Aktivkohle enthält.
Vorrichtung nach Anspruch 1 oder 2

6. Vorrichtung nach Anspruch 1 oder 2 wobei die Porenweite des Membranfilters die Passage von Peptiden und Proteinen bis zu einem Molekülgewicht von 70.000 Dalton erlaubt und dass der Regenerationskreislauf mindestens, aber nicht unbedingt ausschließlich einen Adsorber auf Basis von Aktivkohle enthält, deren Perfusionskanäle mindestens 10 nm, maximal aber 100 um weit sein dürfen, die durch geringe Diffusionsstrecken langsam diffundierende Moleküle besser entfernen.

7. Vorrichtung nach Anspruch 1 oder 2 wobei
die Porenweite des Membranfilters die Passage von Arginin erlaubt und der Regenerationsgreislauf mindestens, aber nicht unbedingt ausschließlich einen Adsorber auf Basis von Aktivkohle enthält.

8. Vorrichtung nach Anspruch 1 oder 2 wobei
die Porenweite des Membranfilters die Passage von Interleukinen der Gruppe 1 erlaubt und dass der Regenerationskreislauf einen Adsorber auf Basis von Aktivkohle enthält, deren Perfusionskanäle mindestens 10 nm, maximal aber 100 um weit sein dürfen, die durch geringe Diffusionsstrecken langsam diffundierende Moleküle besser entfernen und/oder einen Membranfilter mit einem Rückhaltevermögen für Interleukin 1.

## Claims

1. Device for conserving diafiltrate in a dialysis device, consisting of a regeneration loop, pump and filter, **characterized in that**
a diafiltrate tube collecting the fluid of a rinse solution compartment (B) from a filter has arranged thereon a 3-way connector through which a sub-stream of the diafiltrate is connected to a regeneration loop (RL) via a separate pump in such a way that the diafiltrate is split into two streams, one stream containing substance group X being discarded and the other stream containing substance group Y being recycled via the regeneration loop (RL), and **in that** the substance group Y is admixed again with the fresh diafiltrate via a 3-way connector.

2. Device according to Claim 1, **characterized in that** net dialysate and/or net substituate is admixed.

3. Device according to Claim 1 or 2, wherein the regeneration loop contains at least one activated carbon-based adsorber, but not necessarily exclusively said adsorber.

4. Device according to Claim 1 or 2, wherein the regeneration loop contains at least one membrane filter, but not necessarily exclusively said filter, which membrane filter removes substances from the RL through a smaller pore size than the membrane filter processing a fluid compartment (A), said substances having previously been filtered from the fluid compartment (A) into the diafiltrate.

5. Device according to Claim 1 or 2, wherein the pore size of the membrane filter allows the passage of glutamine and the regeneration loop contains at least one activated carbon-based adsorber, but not necessarily exclusively said adsorber.

6. Device according to Claim 1 or 2, wherein the pore size of the membrane filter allows the passage of peptides and proteins up to a molecular weight of 70 000 daltons and wherein the regeneration loop contains at least one activated carbon-based adsorber, but not necessarily exclusively said adsorber, whose perfusion channels must be at least 10 nm, but not more than 100 µm, long, which perfusion channels are better at removing slowly diffusing molecules as a result of small diffusion distances.

7. Device according to Claim 1 or 2, wherein the pore size of the membrane filter allows the passage of arginine and the regeneration loop contains at least one activated carbon-based adsorber, but not necessarily exclusively said adsorber.

8. Device according to Claim 1 or 2, wherein the pore size of the membrane filter allows the passage of interleukins of the interleukin 1 family and wherein the regeneration loop contains an activated carbon-based adsorber whose perfusion channels must be at least 10 nm, but not more than 100 µm, long, which perfusion channels are better at removing slowly diffusing molecules as a result of small diffusion distances, and/or a membrane filter having a retention capacity for interleukin 1.

## Revendications

1. Dispositif servant à économiser du diafiltrat dans un dispositif de dialyse, le dispositif étant constitué d'un circuit de régénération, de pompes et de filtres, **caractérisé en ce que**
un raccordement à trois voies est disposé sur un conduit flexible à diafiltrat qui collecte le liquide provenant d'un filtre d'un compartiment (B) de solution de rinçage,
**en ce que** le raccordement à trois voies relie un écoulement partiel de diafiltrat à un circuit de régénération (RKL) par l'intermédiaire d'une pompe séparée,
**en ce que** le diafiltrat est divisé en deux écoulements dont l'un qui contient le groupe X de substances est rejeté et l'autre qui contient le groupe Y de substances est réutilisé par l'intermédiaire du circuit de régénération (RKL) et
**en ce que** le groupe Y de substances est mélangé de nouveau avec le diafiltrat frais par l'intermédiaire d'un raccordement à trois voies.

2. Dispositif selon la revendication 1, **caractérisé en ce que** le dialysat net et/ou le substituant net sont mélangés.

3. Dispositif selon les revendications 1 ou 2, dans lequel le circuit de régénération contient au moins un et éventuellement plusieurs absorbeurs à base de charbon actif.

4. Dispositif selon les revendications 1 ou 2, dans lequel le circuit de régénération contient au moins un et éventuellement plusieurs filtres à membrane qui enlèvent des substances du RKL par des pores d'une taille plus petite que ceux du filtre à membrane qui traite un compartiment (A) à liquide, ces substances ayant été séparées par filtration du diafiltrat provenant du compartiment (A) à liquide.

5. Dispositif selon les revendications 1 ou 2, dans lequel la taille des pores du filtre à membrane permet le passage de la glutamine et en ce que le circuit de régénération contient au moins un et éventuellement plusieurs absorbeurs à base de charbon actif.

6. Dispositif selon les revendications 1 ou 2, dans lequel la taille des pores du filtre à membrane permet le passage de peptides et de protéines jusqu'à un poids moléculaire de 70 000 Dalton et en ce que le circuit de régénération contient au moins un et éventuellement plusieurs absorbeurs à base de charbon actif dont les canaux de perfusion peuvent être d'au moins 10 nm, mais d'au plus 100 µm et éliminent mieux par de petits parcours de diffusion les molécules diffusant lentement.

7. Dispositif selon les revendications 1 ou 2, dans lequel la taille des pores du filtre à membrane permet le passage de l'arginine et le circuit de régénération contient au moins un et éventuellement plusieurs absorbeurs à base de charbon actif.

8. Dispositif selon les revendications 1 ou 2, dans lequel la taille des pores du filtre à membrane permet le passage d'interleukines du groupe 1 et en ce que le circuit de régénération contient un absorbeur à base de charbon actif dont les canaux de perfusion peuvent être d'au moins 10 nm mais au plus de 100 µm, qui éliminent mieux par de petits parcours de diffusion les molécules diffusant lentement et/ou un filtre à membrane qui présente une capacité de retenue de l'interleukine 1.
